# EUROPÄISCHE PATENTANMELDUNG

(11) **EP 0 787 985 A1**
(43) Veröffentlichungstag der Anmeldung: **06.08.1997**
(21) Anmeldenummer: 96101563.3
(22) Anmeldetag: 03.02.1996
(51) Int. Cl.: G01N 27/62

(54) **Verfahren und Vorrichtung für den Nachweis von organischen Dämpfen und Aerosolen**

(71) Anmelder: CERBERUS AG, CH-8708 Männedorf (CH)
(72) Erfinder: Loepfe, Markus, Dr., CH-8002 Zürich (CH); Wieser, Dieter, CH-8008 Zürich (CH); Ryser, Peter, Dr., CH-8712 Stäfa (CH)

(57) **Zusammenfassung**

Bei einem Verfahren für den Nachweis von organischen Dämpfen und Aerosolen mittels Oberflächenionisation, insbesondere Aminen, Hydrazinen und stickstoffhaltigen Verbindungen, die aus Bränden entstehen, kondensieren die betreffenden Moleküle auf einer leitenden Vorrichtung (4). Diese wird durch Strompulse durch eine resistive Heizung (5) erhitzt, wodurch die kondensierten Moleküle thermisch ionisiert werden. Die erzeugten Ionen werden von einer Kollektorelektrode (9) aufgefangen und der resultierende Ionenstrompuls von einer Transimpedanzschaltung (15) verstärkt. Der Strompuls für die Erhitzung der leitenden Vorrichtung (4) dauert so lange an bis der Ionenstrompuls abgeklungen ist und die leitende Vorrichtung (4) von Restsubstanzen befreit ist. Dadurch bleibt die leitende Vorrichtung frei von Verschmutzung und verfügt über eine längere Lebensdauer. Die zeitgemittelte Leistungsaufnahme des Verfahrens beträgt weniger als 2 mW. Die leitende Vorrichtung (4) weist eine resistive, mäanderförmige Heizung (5) auf die als dünne Schicht auf einer Si₃N₄-Membran (3) aufgebracht ist, die über einer Öffnung (2) eines unterätzten Silizium-Chips (1) liegt. In einer weiteren Ausführungsform der leitenden Vorrichtung (4) ist auf der resistiven Heizung (5) eine durch eine Isolierschicht (7) beabstandete leitende Schicht (8) angeordnet.

## Beschreibung

Die Erfindung betrifft ein Verfahren und eine Vorrichtung für den Nachweis von organischen Dämpfen und Aerosolen mittels Oberflächenionisation. Insbesondere betrifft sie den Nachweis von organischen Dämpfen, Aerosolen und flüchtigen Substanzen, die bei Bränden entstehen.

Organische Dämpfe und Aerosole, die bei Bränden entstehen, werden durch verschiedene bekannte Verfahren nachgewiesen, wovon die einen Verfahren in kommerziell erhältlichen Rauchdetektoren, andere in Labormessgeräten angewendet werden. Unter den kommerziell erhältlichen Rauchdetektoren sind die sogenannten Ionisationsrauchdetektoren, bei denen die Luft in einer Ionisationskammer mittels einer radioaktiven Quelle ionisiert wird und die erzeugten Ionen durch ein angelegtes elektrisches Feld als Ionenstrom gemessen werden. Die Anwesenheit von Aerosolen in der Ionisationskammer bewirkt eine Verringerung des Ionenstroms. In weiteren kommerziellen Rauchdetektoren sowie in Messgeräten werden Aerosole und Dämpfe durch optische Nachweisverfahren nachgewiesen, wie zum Beispiel durch Messung der optischen Transmission, der spektralen Absorption oder von an Aerosolen gestreutem Licht. Ein weniger angewandtes Verfahren ist der Nachweis mittels Oberflächenionisation, bei dem an einer erhitzten Oberfläche nachzuweisende Moleküle thermisch ionisiert werden und die erzeugten Ionen in einem angelegtem elektrischen Feld als Ionenstrom gemessen werden.

In der Anwendung von solchen Nachweisverfahren ist es oft erwünscht, dass durch das gewählte Verfahren die verschiedenen vorkommenden Dämpfe und Aerosole mit gleicher Empfindlichkeit nachgewiesen werden. Der Nachweis von Aerosolen mittels Ionisationsrauchdetektoren erfolgt in dieser Hinsicht zuverlässig, indem die meisten bei Bränden entstehenden Dämpfe und Aerosole in kurzer Zeit eine Verringerung des Ionenstroms verursachen. Die Notwendigkeit einer radioaktiven Quelle zur Erzeugung des Ionenstroms ist jedoch bei der kommerziellen Anwendung mit Schwierigkeiten verbunden, wie zum Beispiel die der Entsorgung von radioaktiven Abfällen, die bei der Herstellung und Wartung dieser Detektoren entstehen.

Das optische Nachweisverfahren durch die Messung von an Aerosolen gestreutem Licht ist für die verschiedenen bei Bränden entstehenden Aerosole verschieden empfindlich. Während hellfarbige Aerosole einfallendes Licht stark streuen, streuen dunkelfarbige Aerosole das einfallende Licht im Verhältnis weniger. Die Lichtstreuung ist also von der Art des Aerosols abhängig.

Eine Messung der optischen Transmission ist in Hinsicht auf einen gleichmässigen Nachweis verschiedener Aerosoltypen erfolgreicher, ist aber in der Ausführung, insbesondere beim Nachweis kleinerer Konzentrationen problematisch. Ein Nachweis kleiner Konzentrationen verlangt entweder eine Messung über eine lange Messstrecke oder, wenn nur eine kurze Messstrecke vorhanden ist, eine Messung mit sehr hoher Empfindlichkeit. Beide dieser Messmethoden erweisen sich als aufwendig und nur beschränkt anwendbar.

Eine Messung der spektralen Absorption ist meist mit grossen Kostenaufwand verbunden, da spezielle Lichtquellen, Filter und Detektoren notwendig sind. Auch verlangt der Nachweis verschiedener Aerosoltypen mit der gleichen Messvorrichtung ein Auswechseln von Komponenten, wodurch die Anwendung dieser Messmethode auf Labormessgeräte beschränkt ist.

Beim Nachweis von Dämpfen und Aerosolen mittels Oberflächenionisation kondensieren deren Moleküle zunächst an einer leitenden Oberfläche. Zum Teil werden Moleküle auch von der Oberfläche adsorbiert. Durch Erhitzung der Oberfläche auf mehrere hundert Grad Celsius werden die Moleküle ionisiert und die positiven sowie negativen Ionen von der Oberfläche emittiert. Zur Messung der emittierten Ionen werden diese von einer über der Oberfläche angeordneten Gegenelektrode aufgefangen. Je nach der Ladung der Ionen, positiv oder negativ, die nachgewiesen werden sollen, wird die Elektrode auf ein entsprechendes negatives bzw. positives Potential gelegt. Durch die Messung des resultierenden Ionenstroms wird die Anwesenheit von ionisierbaren Molekülen in der Umgebung der Messvorrichtung nachgewiesen.

Ein erfolgreicher Nachweis von Aerosolen mittels Oberflächenionisation wird in erster Linie vom Grad der Ionisierbarkeit der Moleküle bestimmt. Der Grad der Ionisierbarkeit hängt einerseits von der Temperatur der ionisierenden Oberfläche ab, andererseits von dem Partikelionisationspotential (bei positiven Ionen) und der Elektronenaffinität (bei negativen Ionen) im Vergleich zur Austrittsarbeit des Oberflächenmaterials. Positive Ionen bilden sich am besten, wenn das Ionisationspotential tief ist, negative Ionen, wenn die Elektronenaffinität hoch ist.

Substanzen, deren Dämpfe und Aerosole mittels Oberflächenionisation nachweisbar sind, das heisst an einer leitenden Oberfläche ionisierbar sind, sind unter anderen Amine, Hydrazine und stickstoffhaltige Verbindungen wie zum Beispiel Triethylamin, Trimethylamin, Pentylamin, Butylamin, Propylamin, Aminosäuren, Essigsäuren, Phenole, Aniline und Ammoniak. Insbesondere sind die Aerosole und Dämpfe, die aus brennenden Zellstoffen entstehen, durch diese Methode nachweisbar.

In US 4,176,311 wird eine Vorrichtung für den Nachweis von Rauchpartikeln beschrieben. Die Vorrichtung weist einen Draht aus Wolfram oder Platin und eine zylinder- oder halbzylinderförmige Gegenelektrode auf An dem Draht, durch den kontinuierlich ein elektrischer Strom fliesst, sodass seine Temperatur stetig 500 °C beträgt, werden die Rauchpartikel thermisch ionisiert, wodurch ein messbarer Ionenstrom zur Gegenelektrode erzeugt wird. Nachteilig bei dieser Messvorrichtung, insbesondere für eine kommerzielle Anwendung, ist die beschränkte Lebensdauer des Heizdrahtes. Die Empfindlichkeit der Vorrichtung nimmt ausserdem als Funktion der Zeit stetig ab, weil der Draht vermehrt verschmutzt wird. Ferner ist die Leistungsaufnahme durch die kontinuierliche Erhitzung des Drahtes relativ hoch.

Es ist die Aufgabe der vorliegenden Erfindung für den Nachweis mittels Oberflächenionisation von organischen Dämpfen und Aerosolen, insbesondere von Aminen, Hydrazinen und stickstoffhaltigen Verbindungen und Dämpfen und Aerosolen, die aus Bränden entstehen, ein Verfahren und eine Vorrichtung zu seiner Durchführung zu schaffen. Diese sollen die Nachteile des obengenannten Standes der Technik vermeiden, indem das Verfahren eine geringe Leistungsaufnahme erfordert und die Vorrichtung über eine im Vergleich zum Stand der Technik verlängerte Zeitdauer funktionstüchtig und durch ihren Aufbau kostengünstig ist, indem sie in einer Vielzahl auf automatisierte Weise herstellbar und mit elektronischen Bauteilen integrierbar ist.

Die Aufgabe wird durch ein Verfahren gelöst, bei dem ionisierbare Moleküle der nachzuweisenden organischen Dämpfe und Aerosole an einer leitenden Vorrichtung bei Raumtemperatur kondensieren und/oder adsorbieren und durch pulsweise Erhitzung der leitenden Vorrichtung mittels einer resistiven Heizung thermisch ionisiert, von der leitenden Vorrichtung emittiert und auf einer Kollektorelektrode aufgefangen werden und der erzeugte Ionenstrom durch eine Signalaufbereitungsschaltung verstärkt und gemessen wird. Nach jeder Ionenemission wird die leitende Vorrichtung so lange weiter erhitzt bis der Ionenstrom wieder auf den stationären Wert abgefallen ist, der einer Abwesenheit von Molekülen entspricht. Das bedeutet, dass die Vorrichtung ab diesem Zeitpunkt von Restsubstanzen befreit ist und nach ihrer Abkühlung erneut anwesende Moleküle auf ihr kondensieren und nachgewiesen werden. Der Nachweis ist auf diese Weise über eine längere Zeit gewährleistet, da eine Verschmutzung der Vorrichtung vermieden wird. Durch die pulsweise Erhitzung der Vorrichtung erfordert das Verfahren eine geringe über die Zeit gemittelte Leitungsaufnahme. Bei Anwesenheit von ionisierbaren Molekülen hat der durch die Oberflächenionisation herbeigeführte Ionenstrom einen Wert im Grössenbereich von nA, was ungefähr dem tausendfachen des Stroms entspricht, der in reiner Luft gemessen wird.

Die Vorrichtung zur Lösung der Aufgabe enthält eine leitende Vorrichtung mit einer Oberfläche für die Ionenemission und eine Kollektorelektrode zum Auffangen der emittierten Ionen, die über der leitenden Vorrichtung angeordnet ist, wobei die leitende Vorrichtung eine resistive Heizung aufweist, die auf einer Siliziumnitrid-Membran (Si₃N₄) aufgebracht ist, welche über einer Öffnung eines unterätzten Silizium-Chips liegt. In einer weiteren Ausführung der leitenden Vorrichtung ist auf der resistiven Heizung eine durch eine Isolierschicht beabstandete leitende Schicht angeordnet.

Die resistive Heizung besteht aus einem dünnschichtigen Mäander aus einem Edelmetall, wie zum Beispiel Platin, der durch Anlegen von Strompulsen erhitzt wird. Durch die Strompulse wird dabei eine sehr hohe Heizrate erzielt. In der ersten Ausführung der leitenden Vorrichtung dient die resistive Heizung zur Erhitzung und zugleich als Oberfläche zur Ionenemission. Die starke Erhitzung bewirkt eine Ionisierung von an der Oberfläche adsorbierten Moleküle sowie die Emission der erzeugten Ionen. Bei der zweiten Ausführung der leitenden Vorrichtung dient eine zusätzliche leitende Schicht als Oberfläche zur Ionenemission und die resistive Heizung zu ihrer Erhitzung. Durch eine dünne Schicht elektrisch isolierenden Materials ist die ionenemittierende leitende Schicht von der resistiven Heizung getrennt. Im Vergleich zur ersten Ausführung der leitenden Vorrichtung bewirken die leitende Schicht mit dazwischen liegenden isolierender Schicht eine gleichmässigere Erhitzung der ionenemittierenden Oberfläche.

Die leitende Schicht, die Isolierschicht, die resistive Heizung und die Si₃N₄-Membran werden als dünne Schichten unter Verwendung von Standard-Chipherstellungstechnik, wie zum Beispiel Masken- und Vaküumdünnschichttechnik, auf ein Standard-Silizium-wafer aufgetragen, sodass in einem automatisierten Herstellungsdurchgang eine Vielzahl der Vorrichtungen produziert werden. Indem die Vorrichtung auf einem Silizium-Chip angeordnet ist, sind die elektrischen Bauteile, die für die Signalaufbereitung zur Messung des Ionenstroms erforderlich sind, auf demselben Silizium-Chip integriert. Die Elektronik wird in dieser integrierten Form auch auf automatisierte Herstellweise auf den Chip aufgebracht.

Die pulsweise Erhitzung der leitenden Vorrichtung bewirkt zunächst eine Ionenemission in der Form eines Ionenpulses, der zuerst steil ansteigt und dann langsam abfällt. Die Spannung über der Heizung wird über die gesamte Dauer des resultierenden Ionenpulses konstant gehalten, sodass die leitende Vorrichtung und die ionenemittierende Oberfläche auch nach Erreichung des Maximums des Ionenpulses erhitzt bleibt. Während dieser Zeit werden auf der ionenemittierenden Oberfläche übrigbleibende Substanzen durch Verbrennung, katalytische Verbrennung, Verdampfung oder Desorption von ihr gelöst bis der Ionenpuls auf einen stationären Wert abgefallen ist. Sobald die Oberfläche von Restsubstanzen befreit ist und keine Spannung mehr über der Heizung angelegt ist, kühlt sie sich ab, und es erfolgt eine erneute Kondensierung von Molekülen. Die wiederholte Befreiung der Oberfläche von Restsubstanzen vermeidet ihre Verschmutzung und gewährleistet somit eine verlängerte Funktionsdauer der Vorrichtung.

Ausführungsformen des Verfahrens und der Vorrichtung zu seiner Durchführung werden anhand der Figuren 1 bis 6 näher erläutert.

Fig. 1 a) und b) zeigen stark vergrössert einen Querschnitt durch den schichtweisen Aufbau der ersten bzw. zweiten Ausführung der leitenden Vorrichtung.

Fig. 2 zeigt eine Draufsicht der leitenden Vorrichtung von Fig. 1 b).

Fig. 3 zeigt eine Seitenansicht der Vorrichtung mit der Kollektorelektrode zum Auffangen der Ionen.

Fig. 4 zeigt eine Draufsicht der Vorrichtung mit der dazugehörigen elektrischen Schaltung zur Signalaufbereitung in einem Standardgehäuse für integrierte Chips (IC) angeordnet.

Fig. 5 zeigt ein elektrisches Schaltschema für das Verfahren.

Fig. 6 a) - e) zeigt den zeitlichen Ablauf der Betriebssignale der Vorrichtung und der resultierenden Ionenstromsignale.

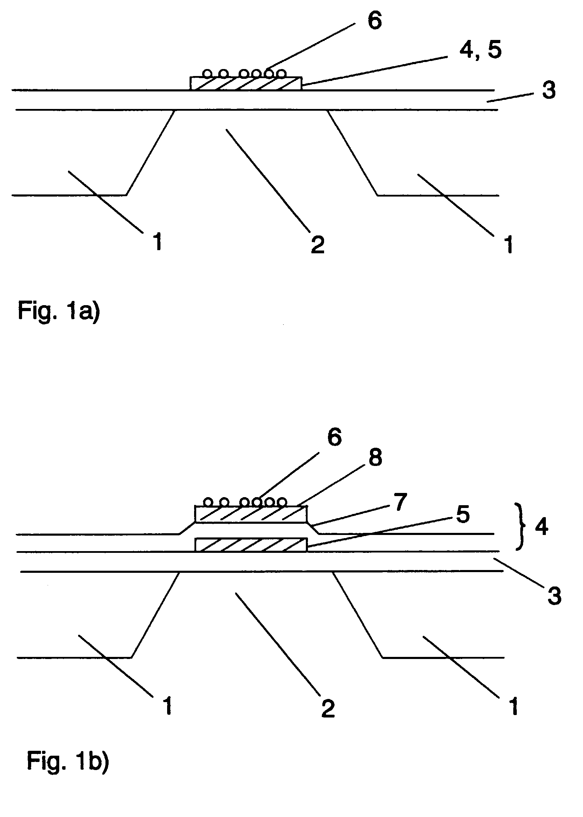

Fig. 1 a) zeigt einen Silizium-Chip 1, der in seiner Mitte eine rechteckige Öffnung 2 aufweist, die durch Auftragen einer Schicht 3 von Si₃N₄ und selektives Ätzen des Siliziums herbeigeführt worden ist. Die Si₃N₄-Schicht 3 wirkt über der Öffnung 2 als dünne Membran 3 und dient als Trägerschicht für die leitende Vorrichtung 4. Die Dicke der Si₃N₄-Membran 3 beträgt beispielsweise 0.6 µm. Auf der Si₃N₄-Membran 3 liegt eine zweite (schraffierte) Schicht 5, die resistive Heizung 5, deren Flächenausdehnung etwas kleiner als die Öffnung 2 im Silizium-Chip 1 ist. Sie besteht aus einem Material, das sich resistiv erhitzt und beim Aufbringen einer dünnen Schicht gut auf dem Si₃N₄ haftet. Hierzu eignen sich Edelmetalle wie Platin. Die Schicht 5, die resistive Heizung 5, ist in der Form eines Mäanders aufgetragen, dessen Enden elektrische Kontaktflächen aufweisen. Auf der resistiven Heizung 5 sind auf ihr kondensierte und/oder adsorbierte Moleküle 6 gezeigt.

Fig. 1 b) zeigt eine zweite Ausführung der leitenden Vorrichtung mit einer elektrisch isolierenden Schicht 7, welche die resistive Heizung 5 über den Bereich der Öffnung 2 hinaus überdeckt. Sie besteht zum Beispiel aus einer Schicht von Si₃N₄ oder zwei Schichten übereinander von Si₃N₄ und SiO₂. Die Schicht 7 isoliert die resistive Heizung 5 von einer (schraffierten) leitenden Schicht 8, auf deren Oberfläche Ionen erzeugt und emittiert werden. Die leitende Schicht 8 hat die gleiche Flächenbreite wie die resistive Heizung 5, und besteht aus einem mindestens leicht leitenden Material. Vorzugsweise besteht sie aus Platin, da dieses Metall erfahrungsgemäss für die Oberflächenionisation geeignet ist. Eventuell eignen sich auch Halbleitermaterialien. Auf der leitenden Schicht 8, die sich zwischen den Heizpulsen abkühlt, sind wiederum kondensierte Moleküle 6 gezeigt.

Der Silizium-Chip 1 hat, wie Standard-Silizium-wafers, eine Dicke von 0.38 mm. Die daraufliegenden Schichten 3, 5, 7 und 8 haben jeweils eine Dicke von einigen 100 Nanometern. Sind für die Schichten andere Materialen gewählt, sind je nach Material andere entsprechende Schichtdicken mehr geeignet und Haftschichten zwischen den Schichten erforderlich, um eine genügende Haftung zu gewährleisten.

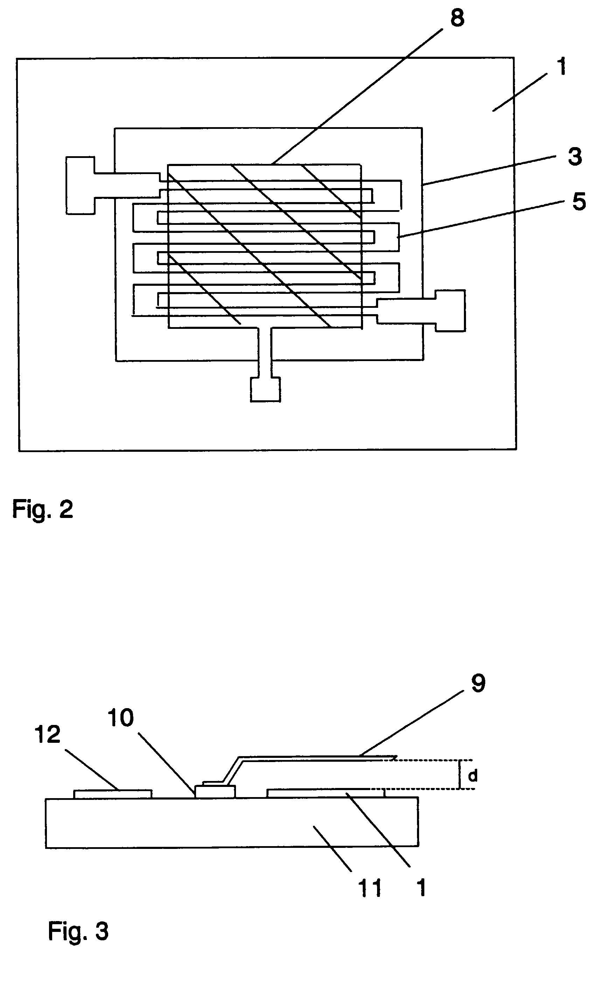

Fig. 2 zeigt eine Draufsicht der leitenden Vorrichtung von Fig. 1 b) mit dem Silizium-Chip 1, der Si₃N₄-Membran 3 und der mäanderförmigen resistiven Heizung 5 mit elektrischen Kontaktflächen zu ihrer Betreibung. Über der Heizung 5 liegt (schraffiert) die ionenemittierende, leitende Schicht 8 mit einer elektrischen Kontaktfläche. (Zwecks der einfacheren Demonstration ist die elektrisch isolierende Schicht 7 hier nicht eingezeichnet). Die Heizung 5 erhitzt die leitende Schicht 8 möglichst gleichmässig über ihre ganze Fläche. Hierzu sind die leitenden Bahnen der Heizung in der Form eines Mäanders möglichst dicht über die ganze Fläche gezogen. Bei den 180°-Kurven des Mäanders ist die Bahnbreite jeweils grösser als bei den geraden Strecken. Dadurch ist die Heizleistung bei den Kurven, die ausserhalb der leitenden Schicht 8 liegen, geringer als bei den geraden Strecken, die unter ihr liegen. Die Bahnbreite beträgt bei den geraden Strecken des Mäanders ca. 40 µm, bei den Kurven ca. 250 µm.

Die Seitenansicht der Vorrichtung in Fig. 3 zeigt den Silizium-Chip 1 mit den oben erwähnten Schichten 3, 5, 7 und 8. In der Form eines Bügels ist über dem Silizium-Chip 1 eine Kollektorelektrode 9 angeordnet. Der Fuss des Bügels ist auf einer mit leitendem Material beschichteten Hilfssubstrat 10 befestigt. Durch die leitende Schicht des Hilfssubstrats 10 wird eine Spannung zum Auffangen der Ionen angelegt. Werden positive Ionen von der Oberfläche der leitenden Vorrichtung emittiert, beträgt bei einer Distanz d von 0.8 mm zwischen der leitenden Vorrichtung und der Kollektorelektrode 9 die Spannung an der Kollektorelektrode 9 beispielsweise - 100 Volt und die an der leitenden Vorrichtung um 0 Volt. (Bei der ersten Ausführung der leitenden Vorrichtung ist die an ihr angelegte Spannung gleich der für ihre Erhitzung angelegten Spannung; bei der zweiten Ausführung beträgt die an der leitenden Schicht 8 angelegte Spannung 0 Volt). Je nach Distanz d und gewünschter elektrischen Feldstärke wird die Spannung entsprechend gewählt. Der Silizium-Chip 1 und das Hilfssubstrat 10 liegen auf einer leitenden, vorzugsweise vergoldeten, Fläche 11, auf der die Signalaufbereitungsschaltung 12 angeordnet ist. Anstelle einer diskreten Montage der Signalaufbereitungsschaltung 12 ist auch ihre Integration auf dem Silizium-Chip 1 möglich.

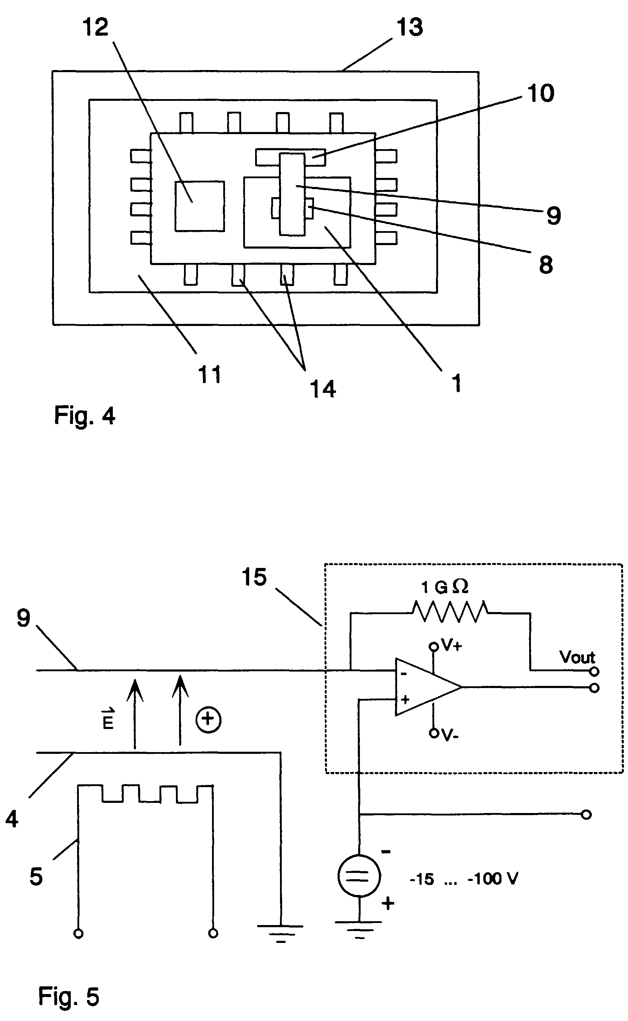

Fig. 4 zeigt die Anordnung der Vorrichtung in einem kommerziellen IC-Gehäuse 13. Die elektrischen Kontaktflächen 14 dienen zur Betreibung der Signalaufbereitungsschaltung 12, der Heizung 5 und zum Anlegen der Spannung an der Kollektorelektrode 9. Die leitende, vergoldete Fläche 11 liegt auf dem isolierenden Material des IC-Gehäuses 13.

Das elektrische Schaltschema in Fig. 5 zeigt den Schaltkreis für die resistive Heizung 5, die auf der leitenden Vorrichtung 4 die Emission von positiven Ionen erwirkt. Die Ionen werden von der Kollektorelektrode 9 aufgefangen und der Ionenstrom durch die Transimpedanzschaltung 15 verstärkt und gemessen. Ein typischer Ionenstrom bei Anwesenheit von ionisierbaren, organischen Dämpfen und Aerosolen hat die Grössenordnung von nA, und eine Verstärkung um einen Faktor von 10⁹ V/A ergibt Messwerte im Voltbereich. Der Operationsverstärker der Transimpedanzschaltung 15 besitzt zur Unterdrückung von Leckströmen einen hohen Eingangswiderstand in der Grössenordnung von 10¹⁵ Ω. Eine angelegte Vorspannung von - 15 bis - 100 Volt bewirkt das notwendige elektrische Feld für die Lenkung der emittierten Ionen auf die Kollektorelektrode 9.

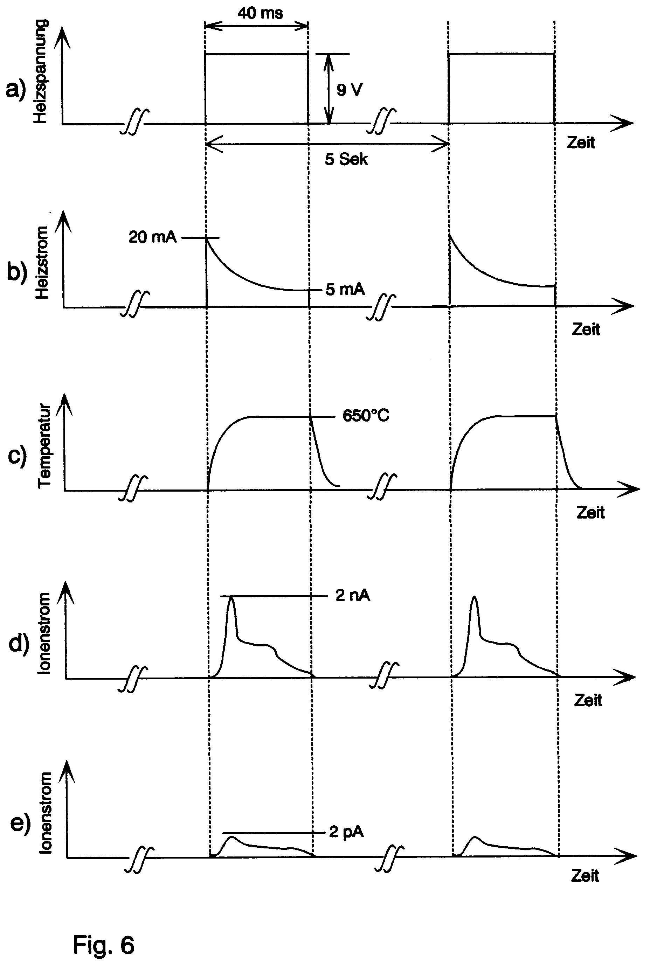

Fig. 6 a) zeigt den zeitlichen Verlauf der an der resistiven Heizung angelegten Spannung. Die Heizung wird beispielsweise durch Pulse von 9 Volt von einer Zeitdauer von 40 ms alle 5 Sekunden betrieben. Bei dieser pulsweisen Erhitzung der leitenden Vorrichtung beträgt die erforderliche zeitgemittelte Leistungsaufnahme weniger als 2 mW. Fig. 6 b) zeigt den resultierenden durch die Heizung fliessenden Strompuls, und Fig. 6 c) zeigt eine Schätzung des Temperaturverlaufs der Heizung. Innerhalb von ca. 10 ms wird eine Temperatur von ca. 650° erreicht, womit eine Heizrate von ca. 60 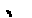 000°C/s erzielt wird. Nach Ende der 40 ms fällt die Temperatur innerhalb von ca. 10 ms wieder auf Raumtemperatur ab. Fig. 6 d) zeigt das Ionenstromsignal, das die Anwesenheit von Aerosolen nachweist, die auf der leitenden Vorrichtung kondensiert sind. Das Beispiel zeigt den Nachweis von Zigarettenrauch. Kurz nach Beginn des Strompulses werden Ionen von der Oberfläche der leitenden Vorrichtung emittiert und ein Ionenstrom ist während einiger ms messbar, der zunächst steil ansteigt und nach Erreichen seines Maximums von 2 nA langsam abklingt. Während des Abklingens des Ionenstrompulses verbleibt die Spannung zur Heizung bis der Ionenstrompuls auf den stationären Wert des Ionenstroms zurückgekehrt ist. Während dieser Zeit wird die Oberfläche der leitenden Vorrichtung durch die andauernde Erhitzung von restlichen Substanzen befreit. Nach Ende des Strompulses bis zum Beginn des nächsten ist keine Spannung angelegt, sodass die Oberfläche sich abkühlt und neue Moleküle an ihr kondensieren. Fig. 6 e) zeigt einen typischen Ruhestrom, das Ionenstromsignal bei reiner Luft, von ca. 2 pA.

## Patentansprüche

1. Verfahren für den Nachweis organischer Dämpfe und Aerosole mittels Oberflächenionisation, wonach ionisierbare Moleküle der nachzuweisenden Dämpfe und Aerosole an einer leitenden Vorrichtung (4) kondensieren und/oder adsorbieren, nach Erhitzung der leitenden Vorrichtung (4) an ihr thermisch ionisiert und die erzeugten Ionen emittiert und von einer Kollektorelektrode (9) aufgefangen werden und der resultierende Ionenstrom von einer Signalaufbereitungsschaltung (12) verstärkt und gemessen wird, dadurch gekennzeichnet, dass die leitende Vorrichtung (4) zur Ionisation der Dämpfe und Aerosole durch Strompulse durch eine resistive Heizung (5) erhitzt wird, wodurch ein Ionenstrompuls erzeugt wird, der von der Signalaufbereitungsschaltung (12) verstärkt und gemessen wird.

2. Verfahren nach Patentanspruch 1, dadurch gekennzeichnet, dass die leitende Vorrichtung (4) durch den Strompuls bei einer Heizrate von mehr als 10 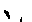 000 °C pro Sekunde erhitzt wird.

3. Verfahren nach Patentanspruch 2, dadurch gekennzeichnet, dass ein Strompuls zur Erhitzung der leitenden Vorrichtung (4) so lange andauert, bis der erzeugte Ionenstrompuls wieder auf einen stationären Wert abgefallen ist, während dessen die leitende Vorrichtung (4) von Restsubstanzen befreit wird, und nach Ende des Strompulses die leitende Vorrichtung (4) sich abkühlt und neue organische Dämpfe und Aerosole bei Raumtemperatur an ihr kondensieren und/oder adsorbieren.

4. Verfahren nach Patentanspruch 3, dadurch gekennzeichnet, dass das Verfahren eine zeitgemittelte Leistungsaufnahme von weniger als 2 mW hat.

5. Verfahren nach Patentanspruch 4, dadurch gekennzeichnet, dass die leitende Vorrichtung (4) alle 5 Sekunden während mindestens 30 Millisekunden durch Anlegen einer Spannung erhitzt wird.

6. Verfahren nach Patentanspruch 5, dadurch gekennzeichnet, dass die organischen Dämpfe und Aerosole Amine, Hydrazine, stickstoffhaltige Verbindungen und/oder Dämpfe und Aerosole, die aus Bränden entstehen, sind.

7. Vorrichtung zur Durchführung des Verfahrens nach Patentanspruch 1 mit einer leitenden Vorrichtung (4) und einer über ihr angeordneten Kollektorelektrode (9), dadurch gekennzeichnet, dass die leitende Vorrichtung (4) eine resistive Heizung (5) aufweist, die als dünne Schicht auf einer über einer Öffnung (2) eines unterätzten Silizium-Chips (1) liegenden Membran angeordnet ist.

8. Vorrichtung nach Patentanspruch 7, dadurch gekennzeichnet, dass die Membran (3) aus Si₃N₄ besteht.

9. Vorrichtung nach Patentanspruch 8, dadurch gekennzeichnet, dass die resistive Heizung (5) die Form eines dünnschichtigen Mäanders aufweist, der die Fläche der zu erhitzenden leitenden Vorrichtung (4) gleichmässig durchläuft.

10. Vorrichtung nach Patentanspruch 9, dadurch gekennzeichnet, dass die resistive Heizung (5) aus einem Edelmetall besteht.

11. Vorrichtung nach Patentanspruch 10, dadurch gekennzeichnet, dass auf der resistiven Heizung (5) eine durch eine elektrisch isolierende Schicht (7) beabstandete leitende Schicht (8) angeordnet ist.

12. Vorrichtung nach Patentanspruch 11, dadurch gekennzeichnet, dass die leitende Schicht (8) aus Metall oder einem Halbleitermaterial besteht.

13. Vorrichtung nach Patentanspruch 12, dadurch gekennzeichnet, dass die elektrisch isolierende Schicht (7) aus Si₃N₄ und/oder SiO₂ besteht.

14. Vorrichtung nach den Patentansprüchen 10 und 13, dadurch gekennzeichnet, dass der Silizium-Chip (1), das Hilfssubstrat (10) für die Kollektorelektrode (9) und die Signalaufbereitungsschaltung (12) auf einer leitenden Fläche (11) in einem Standard-IC-Gehäuse (13) angeordnet sind.

15. Vorrichtung nach Patentanspruch 14, dadurch gekennzeichnet, dass die Signalaufbereitungsschaltung (12) auf dem Silizium-Chip (1) integriert ist.
